# EUROPEAN PATENT SPECIFICATION

(11) **EP 2 462 434 B1**
(45) Date of publication and mention of the grant of the patent: **13.12.2017**
(21) Application number: 09787826.8
(22) Date of filing: 04.08.2009
(51) Int. Cl.: G01N 33/00, F24F 11/00

(54) **APPARATUS FOR DETERMINING THE RESIDUAL QUANTITY OF POLLUTING AGENTS IN A GAS MIXTURE AND PROCESS FOR DETERMINING SUCH QUANTITY**
VORRICHTUNG ZUR BESTIMMUNG DER RESTMENGE AN VERUNREINIGUNGEN IN EINEM GASGEMISCH UND VERFAHREN ZUR BESTIMMUNG EINER DERARTIGEN MENGE
APPAREIL POUR DÉTERMINER LA QUANTITÉ RÉSIDUELLE D'AGENTS POLLUANTS DANS UN MÉLANGE GAZEUX ET PROCÉDÉ POUR DÉTERMINER CETTE QUANTITÉ

(43) Date of publication of application: 13.06.2012
(73) Proprietor: Bitossi, Marco, 50056 Montelupo Fiorentino (Fl) (IT)
(72) Inventor: BALDI Giovanni, I-50025 Montespertoli Fl (IT); CIONI Andrea, I-50053 Empoli Fl (IT); DAMI Valentina, I-51036 Larciano (PT) (IT)
(74) Representative: Bellomia, Paolo
(86) International application number: PCT/IT2009/000360
(87) International publication number: WO 2011/016061

(56) References cited:
- EP-A1- 1 990 080
- US-A1- 2002 193 064
- US-A1- 2003 082 821
- US-B1- 6 374 662

## Description

### Technical Field of the Invention

The present invention refers to an apparatus for determining the residual quantity of polluting agents in a gas mixture and to the relevant process for determining such quantity.

The protection of people's health, both inside and outside buildings, is presently a serious problem for the solution of which many firms and companies invest heavily also for complying to the European directives intended to improve and sanitize work and dwell environments.

The majority of people spend a significant period of time in closed environments.

Several studies have made evident that the level of pollutants in a closed place may be equal to and even higher than that present outside: actually, to the usual polluting substances coming from the outside, such as nitrogen oxides, sulphur oxides, carbon oxides and subtle dusts, other polluting agents can be added, so-called volatile organic substances (VOS), for example formaldehyde and benzene, which are generated by cleaning products, gas hot plates, smoke and furnitures.

As far as the polluting substances present outside the buildings, particularly dangerous are the nitrogen oxides (NOx) produced by combustion.

High concentrations of VOS and NOx can cause such health problems as allergic reactions or more serious symptoms.

For this reason, there is a great interest in searching a way for improving the quality of the closed environments through, for example, the treatment of internal and external surfaces of the buildings with photocatalytic substances able to cause a reduction of the level of polluting substances present in the air.

### State of the Art

A system of air cleaning presently used is based on the use of photocatalytic mortars or cement paints, that is, mortars containing chemical compounds capable of easily reacting, in the presence of UV radiations, with some pollutants and allowing the latter to be removed by direct absorption.

The exposition of said coatings to UV radiation implies the formation of particles which catalyze oxidation-reduction reactions by transforming the pollutants into chemical species having reduced environmental impact.

A photocatalytic substance usually employed for this purpose is titanium dioxide which, in the presence of UV radiation, is able to photocatalytically destroy the polluting substances by degrading them down to water and CO2.

The studies conducted on such a system for verifying the degree of reduction of polluting agents with the use of titanium dioxide, have resulted in the need of providing a reactor in which the contact between the titanium dioxide and the polluting substances is made to occur in the presence of UV light.

The substances to be analysed and the test methods are many. Each reactor is generally dedicated to the analysis, with a particular method, of only one type of polluting agents.

The reactors are often transparent chambers, mostly made of glass, having a considerable capacity and fed by sample gas mixtures suitably prepared to meet the above said requirements and to be sold in the form of cylinders.

However, the present-day test instruments have proved to be non-sufficiently reliable in terms of isolation of the test chamber inside which interference phenomena may take place between the gas mixture to be analysed and the surrounding environment. Moreover, each instrument is dedicated to one test method only and to the evaluation of an individual polluting substance; this brings about significant costs because of the need of providing a plurality of reactors, each being dedicated to a given substance or to be used with one test method, and of setting up such systems as to make them meet the analysis requirements each time. Document US2003/0082821 discloses an apparatus from which the present invention is a development.

### Detailed Description

In this context, the specific technical object of the present invention is to propose an apparatus for determining the quantity of polluting agents in a gas mixture and the relevant process for determining such quantity, being able to overcome the above indicated drawbacks.

In particular, the object of the present invention is to provide an apparatus according to claim 1, for the determination of the quantity of polluting agents in a gas mixture, allowing the implementation of a plurality of test methods and the assessment of the presence of a plurality of polluting agents.

A further object of the present invention is to provide an apparatus, for the determination of the quantity of polluting agents in a gas mixture, which is easy to use, operate and setup, and is able to give very reliable results.

Finally, it is an object of the present invention to propose a process for the determination of the quantity of polluting agents in a gas mixture, by using the apparatus according to the present invention, and allowing the analysis by the same apparatus of different types of polluting agents with different test methods.

The indicated technical task and the specified objects are substantially achieved by an apparatus for determining the quantity of polluting agents in a gas mixture and a process for the determination of such quantity, comprising the characteristics set forth in one or more of the appended claims.

Further characteristics and advantages of the present invention will appear more clearly from the indicative, and thus non-limiting, preferred, but non-exclusive, embodiment of an apparatus for determining the quantity of polluting agents in a gas mixture, as illustrated in the accompanying drawings, wherein:
Fig. 1 is a schematic view of the apparatus for determining the residual quantity of polluting agents in a gas mixture, according to the present invention;
Fig. 2 is a schematic representation of the internal connections of the apparatus in question and structural parts thereof;
Fig. 3 shows an operation diagram of a valve provided within the apparatus in a first operating position; and
Fig. 4 shows an operation diagram of a valve provided within the apparatus in a second operating position.
With reference to the attached figures, numeral 1 indicates as a whole an apparatus according to the invention for determining the residual quantity of polluting agents in a gas mixture.

In order to evaluate the photocatalytic activity of surfaces treated with nano-particles of titanium dioxide, an apparatus 1 has been designed which comprises a closed environment inside which it is possible to accurately simulate the normal atmospheric conditions, for example the composition of the atmosphere and its degree of relative humidity, in the presence of so-called volatile organic substances (VOS) or in the presence of nitrogen oxides (NOx) which are formed essentially during common combustion processes.

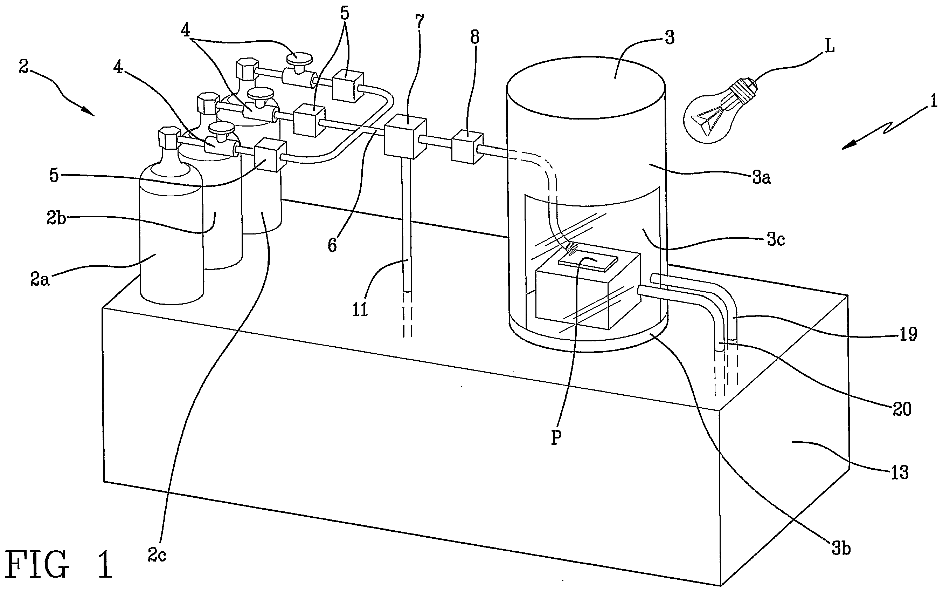

By referring in particular to Fig. 1, the apparatus in question comprises at least one source 2 of controlled air, especially a source of gas mixture with controlled composition.

Advantageously, such source 2 is composed of at least three different types of gas. In this case, there are present a flowing system of moistened air 2a, a system of dry air 2b and a system containing an analyte 2c.

In this way, owing to the adjustment of each of the three flows, it is possible to define the composition of the mixture according to test requirements. Downstream of the source 2 of controlled gas mixture, the apparatus 1 is provided with a reaction chamber or photoreactor 3, inside which the whole quantity of controlled gas mixture is made to transit.

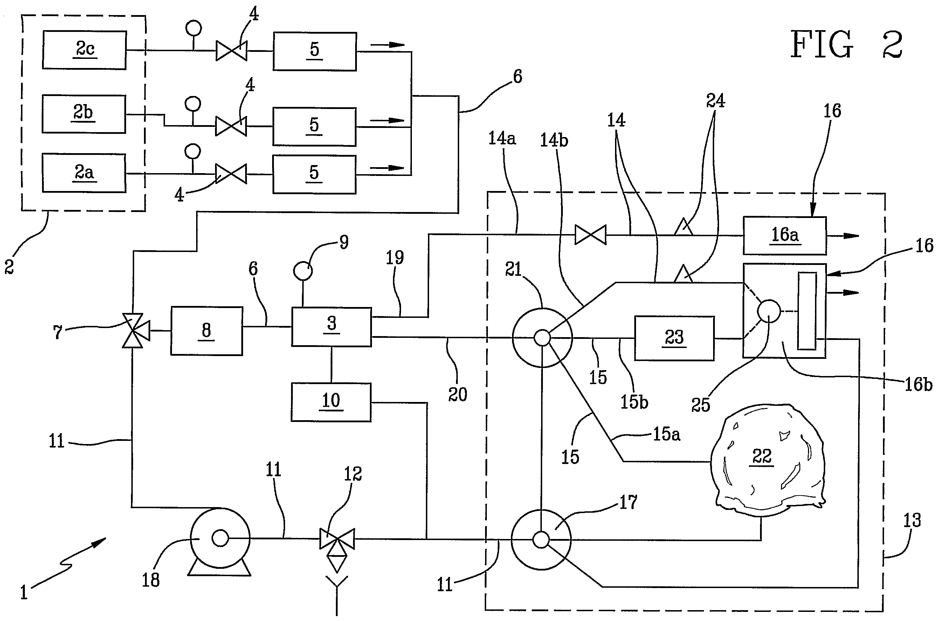

With reference to Fig. 2, coming out of tanks 2a, 2b and 2c of gas-mixture source 2 are respective conduits having therealong respective shutoff valves 4 and relevant instruments 5 for checking the mass flow in each line. The three conduits lead into a single delivery conduit 6 intended to supply the reactor chamber 3.

Provided on the conduit 6 is a three-way valve 7 and, downstream of the latter, an instrument 8 for measuring the flowrate of the gas mixture.

Such instrument 8 allows a useful feedback to be made over each of the instruments which individually control the three flows feeding the whole systems. The reaction chamber 3 is monitored, as for the temperature and relative humidity, by a probe 9 and for the pressure by a regulator 10. In this way, the gas mixture is also controlled in terms of temperature, relative humidity and pressure.

In case of overpressure, the surplus of gas mixture is directed to a return conduit 11 and a three-way relief valve 12, as shown in Fig. 2.

Downstream of the reaction chamber 3, the apparatus 1 exhibits at least one test device 13, wherein the quantity of polluting agents held by the gas mixture which is made to transit through the reaction chamber 3 is assessed.

The test device 13 comprises at least one open-cycle circuit 14 and at least one closed-cycle circuit 15, both interacting with a control unit 16 for a continuous and respectively discontinuous selective determination of the quantity of at least one type of polluting agent held by the gaseous mass leaving the reaction chamber 3.

Advantageously, provision is made for a plurality of open circuits and a corresponding plurality of closed circuits. Preferably, each open circuit makes up, with a respective closed circuit, an operating pair dedicated to the analysis of a given type of polluting agent. Preferably, the apparatus 1 comprises two open circuits 14a, 14b and two closed circuits 15a, 15b.

Advantageously, provision is made for a plurality of control units 16, preferably two units 16a and 16b. Each control unit and/or measuring system 16, dedicated to the analysis of a given type of pollutant, cooperates with a respective pair formed by an open circuit and a closed circuit.

By referring in particular to the diagram of Fig. 2, the apparatus comprises preferably an open circuit 14a, a closed circuit 15a and a respective control unit 16a for a continuous and respectively discontinuous selective determination of the quantity of nitrogen oxides (NOx), and an open circuit 14b, a closed circuit 15b and a respective control unit 16b for a continuous and respectively discontinuous selective determination of the quantity of volatile organic substances (VOS).

In other words, the test device 13, as will be described in detail later on, is able to carry out analysis both continuously, that is, via a disposable open-cycle circuit in which the gas mixture transits only once in the reaction chamber 3, to be discharged afterwards to the outside, and discontinuously, that is, via a closed-cycle circuit allowing the gas mixture to flow more times within the reaction chamber 3.

In the description that follows explicit reference will be made, without any exclusion of generalization, to the preferred embodiment exhibiting two open circuits and two closed circuits for the analysis of NOx and VOS.

Both the closed circuits 15a, 15b are joined to a return conduit 11 via a multiway valve 17. The return conduit 11 fits into the three-way valve 7 for reintroducing the flow of gas mixture into the reaction chamber 3.

Provided on said return conduit 11 is a pump 18 able to recirculate the gas mixture flowing inside the reaction chamber 3.

Along the closed circuit 15a for the analysis of NOx, a tank or pocket 22 is present which is able to store a predetermined quantity of gas mixture for a discontinuous assessment of nitrogen oxides.

Coming out of the reaction chamber 3 is a first conduit 19 which makes the gas mixture flow towards the open circuit 14a for the continuous analysis of NOx.

Moreover, coming out of the reaction chamber 3 is a second conduit 20 which leads into a second multi-way valve 21 communicating with the closed circuit 15a for NOx analysis, with the open circuit 14b for VOS analysis and with closed circuit 15b for VOS analysis.

Provided downstream of the multi-way valve 21 on the closed-cycle circuit 15b for the evaluation of the residual quantity of VOS, is a mass flow-control instrument 23, allowing a control of the composition and flowrate of the gas mixture to be made after each new passage thereof through the reaction chamber 3. The two open-cycle circuits 14a and 14b are instead intercepted by sensors 24 able to monitor the pressure value on the relevant line and also to allow part of the mixture to be discharged, should the pressure exceed a preset value.

The reaction chamber 3 is advantageously made of borosilicate glass and stainless steel. In particular, the chamber 3 is preferably of cylindrical shape formed by joining a cylindrical section 3a made of borosilicate glass with a base 3b made of stainless steel ASI316L, the latter being surface-treated to make its surface chemically inert thereby ensuring an inert and non-porous base. In this way, the chamber results safely isolated from the outside and prevents any risk of pollution of the gas mixture sample flowing through the same chamber 3.

A window 3c made of quartz allows the passage of UV radiation emitted by a light source L, as necessary for the photocatalytic reaction inside the chamber 3. The quartz is preferred to glass for its high transparency to the ultraviolet light ensuring the transmission of the radiation starting from 200 nm. All the metallic parts of the apparatus 1, including the conduits, are preferably made passive by a suitable treatment which cause the surfaces thereof to become inert towards aggressive chemicals.

In use, the reaction chamber 3, interfaced with suitable measuring instruments that constantly monitor the degree of temperature 9, relative humidity 9 and pressure 10 thereinside, is supplied with technical gases, that is, with a properly controlled gaseous mixture.

The gases used for the preparation of the mixture are tapped, as previously mentioned, out of tanks 2a, 2b and 2c which may be cylinders or standard gas-generating systems the concentration of which is exactly known.

By combining the flows entering the system, it is possible to know each time the composition of the desired mixture and to control the flowrate thereof. To this end, each feeding channel coming out from the relevant tank is controlled by an instrument 5 which, owing to its great accuracy, ensures a precise adjustment of the gas flow.

The three flows of gas unite on a single line 6 and, owing to the three-way valve 7, the mixture is conveyed to the reaction chamber 3, also called photoreactor.

Before entering the photoreactor 3, the gas mixture comes across, as previously indicated, a further instrument 8 by which the total flowrate is measured. Positioned inside the photoreactor 3 is an object P whose external surface has been treated beforehand with a photocatalytic substance. In particular, the object P is coated with a titanium dioxide-based substance. The delivery conduit 6 takes the gas mixture to the photoreactor 3 to make the mixture licking constantly the object P.

The gas mixture, in the presence of UV radiation, reacts with the titanium dioxide and, afterwards, leaves the photoreactor 3 to be analysed.

The flow of gas leaving the reaction chamber 3 is controlled each time according to the kind of measurement and process to be evaluated.

This apparatus makes it possible to perform measurements both continually and discontinually on one or more polluting agents. In particular, it is possible to make tests continually and discontinually on the residual quantity of NOx and residual quantity of VOS.

For the evaluation in a continuous-cycle fashion of the residual quantity of NOx, the gas, after having crossed the photoreactor 3, is fed, through the open-cycle circuit 14a, to the control unit 16a which contains chemiluminescence detector for the measurement of NO and NO2 concentration.

The whole quantity of gas mixture emitted by the source 2 goes through the photoreactor 3, is conveyed to the measuring system 16a and discharged from here to the outside.

For the evaluation in a discontinuous-cycle fashion of the residual quantity of NOx, instead, a kinetic test is carried out for establishing the efficiency a sample's photocatalytic activity, by expressing the results in terms of quantity of NOx degraded per unit of surface and time.

At the beginning of each test, a conditioning, that is, a scavenge of the system is carried out by making a quantity of gas mixture presettable synoptically (that is, determined through suitable software) to flow through the system of the apparatus 1 so as to ensure that the composition of the mixture inside the photoreactor 3 is the one desired.

Installed on the closed-cycle circuit 15a used for this method is a tank 22, that is, a pocket having preferably a capacity of about 100 litres and made of a film of material with very low permeability towards the various gases with which the tests are carried out.

The tank 22 has the function of a plenum chamber for storing a certain quantity of gas mixture so as to properly supply it to the control unit 16a and chemiluminescence detector.

Such tank 22 is necessary inasmuch as the reaction chamber 3 has limited dimensions, that is, a rated capacity in the order of 5 litres, but the chemiluminescence detector, instead, requires higher flowrates for the analysis.

Actually, the detector used for determining the NOx, although it has a good sensitivity, inasmuch as it can measure concentrations in the order of parts per milliard, it needs a significant flowrate of gas on input (about 0.6 litres/minute) and times of measurements quite long (about 40 seconds/measurement), especially when approaching the limit of detection capacity of the instrument, that is, when measuring low concentrations of NOx.

The reduced dimensions of the reaction chamber 3, instead, are justified by the fact that, if it is desidered to operate under conditions of fluid's turbulent motion inside the photoreactor 3, as well as in case of dealing with modest flowrates, the dimensions of the system cannot be exceeded, unless to have regions in which the modest flow motion generates phenomena of local lack of homogeneity, as such regions are not acted upon by a suitable turbulence.

As a consequence of the above, it is necessary using a plenum chamber ensuring a store of gas for a good number of analysis to be carried out throughout the process.

The choice of a container with non-rigid walls is justified by the fact that if the gas mixture be withdrawn from a rigid container, there would be variations of pressure and, consequently, also of all the other parameters (relative humidity, temperature, concentration of analytes).

The outlet of the storing pocket 22 is connected to the multiway valve 17 which diverts the flow to the three-way valve 12, which, in turn, allows the flow to be bypassed to either an outlet or a diaphragm pump 18.

Advantageously, the head of pump 18 is made fully of inert material and allows a maximum flowrate of about 15 litres/minute, with a free delivery at atmospheric pressure, and is provided on top with a manual bypass allowing flowrate adjustment.

Such pump 18 has the function of circulating the gas mixture in the closed circuit obtained by switching the three-way valve 7 located upstream of the instrument 8 for the measurement of the gas mixture flowrate.

The filling of the tank 22 is obtained by switching the multiway valve 17 connected downstream of same tank 22 and controlling, with the flowrate-measuring instrument 8 located upstream of the reaction chamber 3, the quantity of gas that fills up the tank 22.

Any pressure increments that could damage the pocket 22 are kept under control by the pressure regulator 10 which brings the pressure back to safety levels by tapping off the surplus of mixture directly from the reaction chamber 3.

Once the system has been conditioned, that is, after the gas mixture has filled completely the system and the tank is full, the test begins: at predetermined times, the multiway valve 21 is switched and the flow is diverted toward the chemiluminescence detector to evaluate the concentration of NOx.

Throughout the test, any variations of temperature and relative humidity are monitored and their value registered.

The completion of the test will allow to have a pattern of the NOx's photodegradation process.

For the continuous analysis of the residual quantity of VOS, instead, use is made of the open-cycle circuit designated by 14b in Fig. 2.

In this case, the gas mixture, after transiting through the photoreactor 3, is fed via the multiway valve 21 to a gas meter held inside the control unit 16b dedicated to the analysis of VOS concentration within the gas mixture.

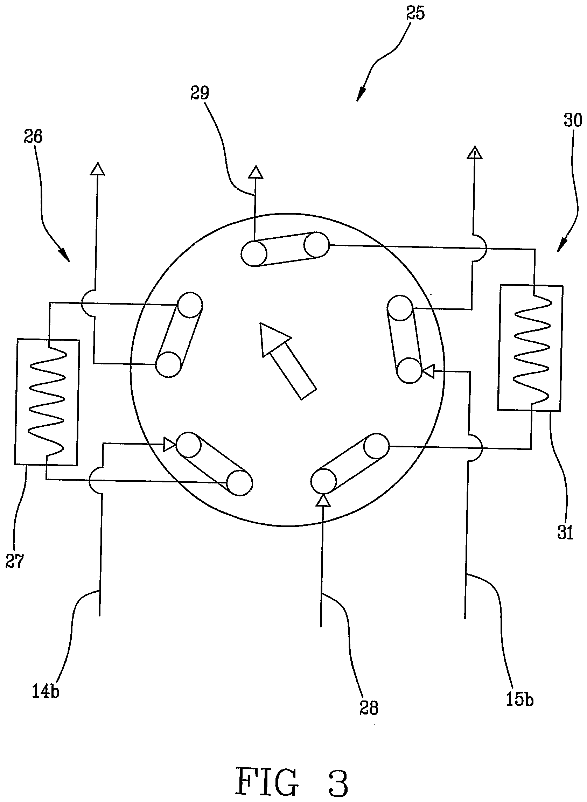

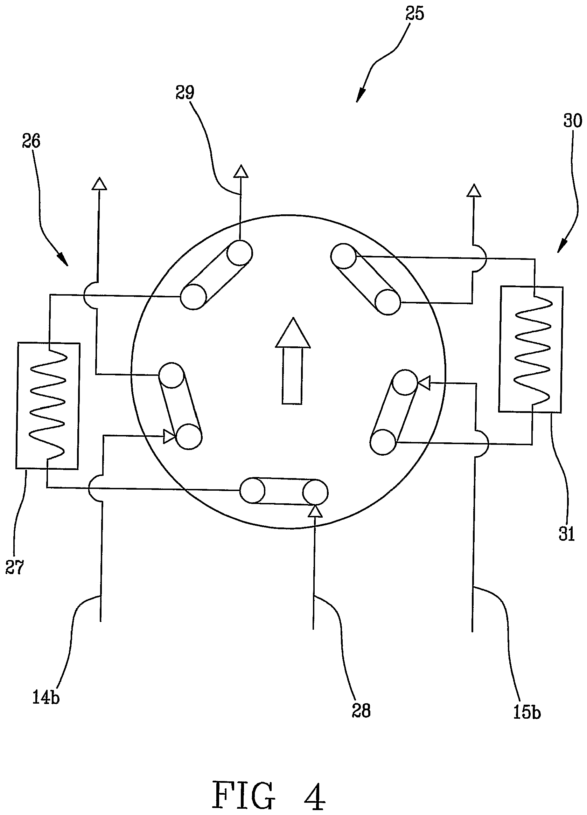

The said unit comprises a ten-way sampling valve 25 whose operating positions are shown in Figs. 3 and 4. The sampling valve 25 is provided with two sampling circuits 26 and 30.

A first circuit 26 is dedicated to the sampling of gases by a continuous cycle analysis.

The gas mixture exiting from the photoreactor 3 flows via conduit 14b into the sampling valve 25 and runs through the first circuit 26 (shown on the left side in Fig. 3) by filling a container 27 with a sample of gas mixture.

When it is desired to carry out the analysis, the sampling valve 25 is switched (Fig, 4) so that the container 27, inside which the sample of mixture is stored, is in fluid communication with other two conduits 28 and 29 through which a carrier gas, such as helium, is made to flow.

The sample of gas mixture is thus conveyed through the conduit 29 to the analysis instrument, a mass spectrometer, the same mixture being pushed by said carrier gas to enter the valve 25 from conduit 28.

For a discontinuous investigation of the VOS photodegradation processes, there is used instead the closed-cycle circuit 15b.

Also in this case the circuit must be properly conditioned as far as to have the desired concentration of gas mixture inside the photoreactor 3.

In this case it is not necessary using a plenum chamber with the gas stored therein inasmuch as the volume of the gas mixture employed for each analysis (about 1 cm3) is significantly less than the rated volume of the system.

As can be shown in the diagram of Fig. 2, the gaseous flow is intercepted by the instrument 23 for controlling the composition and flowrate of the gas mixture after each passage of the latter through the reaction chamber 3.

Such instrument feeds a constant flow of gas to the control unit 16b.

Also in this case, any increases of the pressure upstream of said instrument 23 is monitored by the pressure regulator 10.

Provided inside the control unit 16b is the said sampling ten-way valve 25 which controls again the gas mixture but by using, this time, a second circuit 30 (shown on the right side in Fig. 4).

With reference to Fig. 4, the gas mixture exiting from the photoreactor 3 goes through the sampling valve 25 and fills a new container 31 with a quantity thereof sufficient to perform the analysis.

Once the container 31 is filled up, the valve is switched into the position shown in Fig. 3. Under this condition, the gas stored in the container 31 is conveyed to the mass spectrometer by the carrier gas entering the valve from the conduit 28 and leaving the conduit 29 after having pushed the gas mixture out of the container 31.

By this type of test and, in general, with the discontinuous method of analysis, it is intended to determine the speed with which the VOS or the NOx present in the gas mixture are degraded, by measuring at preset times the variation of concentration of the analyte within the mixture, and by measuring, throughout the duration of the test, the values of temperature and relative humidity.

The measurements made continuously, instead, serve to determine the efficiency of an object P coated with a photocatalytic layer and licked by a constant flow of gas having the same concentration of NOx and VOS all the time throughout the test.

From the above, it is evident that the invention obtains the proposed objects and provides major advantages.

In fact, the described apparatus is able to make analysis both continuously and discontinuously on residual polluting agent present in a gas mixture after the latter has licked an object P coated with photocatalytic material such as titanium dioxide, for example.

In this way, two different methods of investigation are integrated into a single system with remarkable saving of costs and dimensions.

Moreover, the apparatus according to the present invention is able to provide results for more polluting agents, such as NOx and VOS.

Finally, the source of gas mixture, consisting of several supplies of gas, makes it possible to change the composition of the mixture to be analysed according to requirements.

The process of performing the tests of interest by means of the apparatus according to the present invention is efficient and allows to carry out the desired measurements with ease, efficacy and extreme accuracy.

## Claims

1. Apparatus for determining the quantity of polluting agents in a gas mixture comprising a source (2) of a controlled gas mixture, a reaction chamber (3) inside which the said quantity of gas mixture is made to transit, and at least one test device (13) for analysing the quantity of polluting agents held by the gas mixture, **characterized in that** said test device (13) comprises at least one open-cycle circuit (14a, 14b) communicating with the outside, in which the gas mixture transits only once in the reaction chamber (3), to be discharged afterwards to the outside, and at least one closed-cycle circuit (15a, 15b) through which the gas mixture flows more times within the reaction chamber (3); the open-cycle circuit (14a, 14b) and the closed-cycle circuit (15a, 15b) interacting with a respective control unit (16, 16a, 16b) for carrying out respectively a continuous and a discontinuous selective determination of the residual quantity of at least one type of polluting agent held by the gaseous mass.

2. Apparatus according to claim 1, **characterized in that** said test device (13) comprises at least two open-cycle circuits (14a, 15a) and at least two closed-cycle circuit (14b, 15b) for determining the quantity of at least two types of polluting agents; each pair formed of one open-cycle circuit (14a, 14b) and one closed-cycle circuit (15a, 15b) cooperating with a relevant control unit (16, 16a, 16b) capable of determining the residual quantity of a given type of polluting agent.

3. Apparatus according to claim 1 or 2, **characterized in that** said test device (13) comprises a control unit (16a) capable of analysing the residual quantity of nitrogen oxides, and at least one control unit (16b) capable of analysing the residual quantity of volatile organic substances held inside the gaseous mass made to transit through the reaction chamber (3).

4. Apparatus according to claim 3, **characterized in that** it comprises upstream of control unit (16) for controlling the quantity of volatile organic substances, a sampling valve (25) able to selectively operates the open-cycle circuit (14b) and the closed-cycle circuit (15b) dedicated to the analysis of the volatile organic substances.

5. Apparatus according to any of the preceding claims, **characterized in that**, upstream of the reaction chamber (3), an instrument (8) is located for measuring the flowrate of the gas mixture to provide a control of the mixture entering the chamber (3).

6. Apparatus according to any of the preceding claims, **characterized in that** it comprises, on a return conduit (11) located along the closed-cycle circuit (15a, 15b), a pump (18) for pushing the flow of gas mixture into the reaction chamber (3).

7. Apparatus according to any of the preceding claims, **characterized in that** it comprises a plurality of valves (17, 21) for intercepting the flow of controlled gas mixture exiting from the reaction chamber (3).

8. Apparatus according to any of the preceding claims, **characterized in that** said reaction chamber (3) has a quartz window (3c) allowing the passage of light therein so as to facilitate a photocatalytic reaction inside the chamber (3).

9. Process for determining the degree of suppression of polluting agents in a gas mixture, **characterized in that** it comprises the steps of disposing an object (P), coated with a photocatalytic layer, inside a reaction chamber (3) of an apparatus according to any of claims 1 to 8, introducing a controlled gas mixture into the apparatus, illuminating with UV radiation the inside of the reaction chamber (3), filling said reaction chamber (3) with said gas mixture, allowing the gas mixture exiting from the reaction chamber (3) to pass through a test device (13) for the continuous and discontinuous determination of the quantity of polluting agents held by the gas mixture leaving the reaction chamber (3).

10. Process according to claim 9, **characterized by** causing the gas mixture exiting from the reaction chamber (3) to pass inside the test device (13) through an open-cycle circuit (14a) cooperating with a control unit (16a) for the continuous evaluation of nitrogen oxides.

11. Process according to claim 9, **characterized by** causing the gas mixture exiting from the reaction chamber (3) to pass inside the test device (13) through an open-cycle circuit (14b) cooperating with a control unit (16b) for the continuous evaluation of volatile organic substances.

12. Process according to claim 9, **characterized by** causing the gas mixture exiting from the reaction chamber (3) to pass inside the test device (13) through a closed-cycle circuit (15a) cooperating with a control unit (16a) for the discontinuous evaluation of nitrogen oxides.

13. Process according to claim 9, **characterized by** causing the gas mixture exiting from the reaction chamber (3) to pass inside the test device (13) through a closed-cycle circuit (15b) cooperating with a control unit (16b) for the discontinuous evaluation of volatile organic substances.

14. Process according to claim 12, **characterized in that**, before carrying out the discontinuous evaluation of the nitrogen oxides, a step is provided for filling a pocket-like tank (22), disposed on the closed circuit (15a), with a predetermined quantity of controlled gas mixture.

15. Process according to claim 11 or 13, **characterized in that** it comprises the step of causing the gas mixture exiting from the reaction chamber (3) to pass through a sampling valve (25) which directs selectively a suitably removed sample of gaseous mass either toward the open-cycle circuit (14b) or toward the closed-cycle circuit (15b) for the analysis of the residual quantities of volatile organic substances.

## Patentansprüche

1. Vorrichtung zur Bestimmung der Menge an Schmutzstoffen in einem Gasgemisch umfassen eine Quelle (2) eines gesteuerten Gasgemischs, eine Reaktionskammer (3), innerhalb der die Menge des Gasgemischs durchgelassen wird und mindestens ein Prüfgerät (13) für die Analyse der Menge an Schmutzstoffen, die vom Gasgemisch gehalten werden, **dadurch gekennzeichnet, dass** das Prüfgerät (13) mindestens eine Schaltung (14a, 14b) mit offenem Kreislauf umfasst, die mit der Aussenseite kommuniziert, in der das Gasgemisch nur einmal in der Reaktionskammer (3) durchläuft, um anschließen nach aussen abgeführt zu werden, und zumindest eine Schaltung (15a, 15b) mit geschlossenem Kreislauf, durch die das Gasgemisch mehrmals innerhalb der Reaktionskammer (3) fliesst; wobei die Schaltung (14a, 14b) mit offenem Kreislauf und die Schaltung (15a, 15b) mit geschlossenem Kreislauf, die mit einer entsprechenden Steuereinheit (16, 16a, 16b) zum Ausführen von jeweils einer kontinuierlichen und diskontinuierlichen selektiven Bestimmung der Restmenge von zumindest einer Art von Schmutzstoff zusammenwirken, der von der gasförmigen Masse gehalten wird.

2. Vorrichtung nach Anspruch 1, **dadurch gekennzeichnet, dass** das Prüfgerät (13) mindestens zwei Schaltungen (14a, 15a) mit offenem Kreislauf und mindestens zwei Schaltungen (14b, 15b) mit geschlossenem Kreislauf zur Bestimmung der Menge von mindestens zwei Arten von Schmutzstoffen umfasst; wobei jedes Paar, das aus einer Schaltung (14a, 14b) mit offenem Kreislauf und einer Schaltung (15a, 15b) mit geschlossenem Kreislauf besteht, mit einer entsprechenden Steuereinheit (16, 16a, 16b) kooperiert, die in der Lage ist, die Restmenge einer bestimmten Art von Schmutzstoff zu bestimmen.

3. Vorrichtung nach Anspruch 1 oder 2 **dadurch gekennzeichnet, dass** das Prüfgerät (13), eine Steuereinheit (16a), die in der Lage ist, die Restmenge an Stickoxiden zu analysieren, und mindestens eine Steuereinheit (16b) umfasst, die in der Lage ist, die Restmenge der flüchtigen organischen Substanzen zu analysieren, die innerhalb der gasförmigen Masse gehalten werden und die durch die Reaktionskammer (3) durchgelassen werden.

4. Vorrichtung nach Anspruch 3, **dadurch gekennzeichnet, dass** sie stromaufwärts der Steuereinheit (16) für die Steuerung der Menge an flüchtigen organischen Substanzen ein Probenahmeventil (25) umfasst, das in der Lage ist, die Schaltung (14b) mit offenem Kreislauf und die Schaltung (15b) mit geschlossenem Kreislauf, die der Analyse der flüchtigen organischen Substanzen dediziert sind, selektiv zu aktivieren.

5. Vorrichtung nach einem der vorhergehenden Ansprüche, **dadurch gekennzeichnet, dass** stromaufwärts der Reaktionskammer (3), ein Instrument (8) zur Messung der Durchflussgeschwindigkeit des Gasgemischs angeordnet ist, um für die Steuerung des in die Kammer (3) eintretenden Gemischs zu sorgen.

6. Vorrichtung nach einem der vorhergehenden Ansprüche, **dadurch gekennzeichnet, dass** sie auf eine Rücklaufleitung (11), die entlang der Schaltung (15a, 15b) mit geschlossenem Kreislauf angeordnet ist, eine Pumpe (18) zum Schieben der Strömung des Gasgemisches in die Reaktionskammer (3) umfasst.

7. Vorrichtung nach einem der vorhergehenden Ansprüche, **dadurch gekennzeichnet, dass** sie eine Vielzahl von Ventilen (17, 21) zum Abfangen der Strömung des gesteuerten aus der Reaktionskammer (3) austretenden Gasgemisches umfasst.

8. Vorrichtung nach einem der vorhergehenden Ansprüche, **dadurch gekennzeichnet, dass** die Reaktionskammer (3) ein Quarzfenster (3c) aufweiset, um den Durchgang des Lichts darin zu ermöglichen, um eine photokatalytische Reaktion innerhalb der Kammer (3) zu fördern.

9. Verfahren zur Bestimmung des Grades der Unterdrückung von Schmutzstoffen im Gasgemisch, **dadurch gekennzeichnet, dass** es die Schritte des Anordnens eines Objekts (P), der mit einer photokatalytischen Schicht beschichtet ist, innerhalb einer Reaktionskammer (3) einer Vorrichtung nach irgendeinem der Ansprüche 1 bis 8, des Einführens eines gesteuerten Gasgemisches in die Vorrichtung, des Beleuchtens der Innenseite der Reaktionskammer (3) mit UV-Strahlung, Füllens der Reaktionskammer (3) mit dem Gasgemisch, Ermöglichens, dass das Gasgemisch aus der Reaktionskammer (3) austritt, um durch ein Prüfgerät (13) für die kontinuierliche und diskontinuierliche Bestimmung der Menge von Schmutzstoffen durchzulaufen, die vom Gasgemisch beim Verlassen der Reaktionskammer (3) gehalten werden.

10. Verfahren nach Anspruch 9, **dadurch gekennzeichnet, dass** es das Austreten des Gasgemischs aus der Reaktionskammer (3) verursacht, um innerhalb des Prüfgeräts (13) durch eine Schaltung (14a) mit offenem Kreislauf, die mit einer Steuereinheit (16a) für die kontinuierliche Auswertung von Stickoxiden kooperiert, durchzulaufen,

11. Verfahren nach Anspruch 9 **dadurch gekennzeichnet, dass** es das Austreten des Gasgemischs aus der Reaktionskammer (3) verursacht, um innerhalb des Prüfgeräts (13) durch eine Schaltung (14a) mit offenem Kreislauf, die mit einer Steuereinheit (16a) für die kontinuierliche Auswertung der flüchtigen organischen Substanzen kooperiert, durchzulaufen.

12. Verfahren nach Anspruch 9, **dadurch gekennzeichnet, dass** es das Austreten des Gasgemischs aus der Reaktionskammer (3) verursacht, um innerhalb des Prüfgeräts (13) durch eine Schaltung (15a) mit geschlossenem Kreislauf, die mit einer Steuereinheit (16a) für die diskontinuierliche Auswertung von Stickoxiden kooperiert, durchzulaufen.

13. Verfahren nach Anspruch 9, **dadurch gekennzeichnet, dass** es das Austreten des Gasgemischs aus der Reaktionskammer (3) verursacht, um innerhalb des Prüfgeräts (13) durch eine Schaltung (15a) mit geschlossenem Kreislauf, die mit einer Steuereinheit (16a) für die diskontinuierliche Auswertung von flüchtigen organischen Substanzen kooperiert, durchzulaufen.

14. Verfahren nach Anspruch 12, **dadurch gekennzeichnet, dass** vor der Durchführung der diskontinuierlichen Auswertung von Stickoxiden, ein Schritt zum Füllen eines taschenartigen Behalters (22), der auf der geschlossenen Schaltung (15a) platziert ist, mit einer vorgegebenen Menge von gesteuertem Gasgemisch vorgesehen ist.

15. Verfahren nach Anspruch 11 oder 13, **dadurch gekennzeichnet, dass** es den Schritt umfasst, in dem es das Austreten des Gasgemischs aus der Reaktionskammer (3) verursacht, um durch ein Probenahmeventil (25) durchzulaufen, das selektiv eine in geeigneter Weise entfernte Probe der gasförmigen Masse entweder in Richtung der Schaltung (14b) mit offenem Kreislauf oder in Richtung der Schaltung (15b) mit geschlossenem Kreislauf für die Analyse der Restmengen an flüchtigen organischen Substanzen führt.

## Revendications

1. Appareil servant à déterminer la quantité d'agents polluants dans un mélange gazeux comprenant une source (2) d'un mélange gazeux contrôlé, une chambre de réaction (3) à l'intérieur de laquelle ladite quantité de mé-ange gazeux est faite pour transiter, et au moins un appareil de test (13) servant à analyser la quantité d'agents polluants contenue par le mélange gazeux, **caractérisé en ce que** ledit appareil de test (13) comprend au moins un circuit à cycle ouvert (14a, 14b) communiquant avec l'extérieur, dans lequel le mélange gazeux ne transite qu'une fois dans la chambre de réaction (3) pour être déchargé ensuite vers l'extérieur, et au moins un circuit à cycle fermé (15a, 15b) à travers lequel le mélange gazeux s'écoule plusieurs fois dans la chambre de réaction (3) ; le circuit à cycle ouvert (14a, 14b) et le circuit à cycle fermé (15a, 15b) interagissant avec une unité de commande (16, 16a, 16b) respective pour effectuer, respectivement, une détermination sélective continue et discontinue de la quantité résiduelle d'au moins un type d'agent polluant contenu dans la masse gazeuse.

2. Appareil selon la revendication 1, **caractérisé en ce que** ledit appareil de test (13) comprend au moins deux circuits à cycle ouvert (14a, 15a) et au moins deux circuits à cycle fermé (14b, 15b) pour déterminer la quantité d'au moins deux types d'agents polluants ; chaque paire formée d'un circuit à cycle ouvert (14a, 14b) et d'un circuit à cycle fermé (15a, 15b) coopérant avec une unité de contrôle (16, 16a, 12b) correspondante en mesure de déterminer la quantité résiduelle d'un type donné d'agent polluant.

3. Appareil selon la revendication 1 ou 2, **caractérisé en ce que** ledit appareil de test (13) comprend une unité de contrôle (16a) pouvant analyser la quantité résiduelle des oxydes d'azote, et au moins une unité de contrôle (16b) pouvant analyser la quantité résiduelle des substances organiques volatiles contenues à l'intérieur de la masse gazeuse faite pour transiter à travers la chambre de réaction (3).

4. Appareil selon la revendication 3, **caractérisé en ce qu'**il comprend, en amont de l'unité de contrôle (16) servant à contrôler la quantité de substances organiques volatiles, un robinet d'échantillonnage (25) pouvant agir sélectivement sur le circuit à cycle ouvert (14b) et sur le circuit à cycle fermé (15b) dédié à l'analyse des substances organiques volatiles.

5. Appareil selon l'une quelconque des revendications précédentes, **caractérisé en ce que**, en amont de la chambre de réaction (3), se trouve un instrument (8) servant à mesurer le débit du mélange gazeux pour assurer un contrôle du mélange entrant dans la chambre (3).

6. Appareil selon l'une quelconque des revendications précédentes, **caractérisé en ce qu'**il comprend, un conduit de retour (11) situé le long du circuit à cycle fermé (15a, 15b), une pompe (18) servant à pousser le débit du mélange gazeux dans la chambre de réaction (3).

7. Appareil selon l'une quelconque des revendications précédentes, **caractérisé en ce qu'**il comprend une pluralité de vannes (17, 21) servant à intercepter le flux de mélange gazeux contrôlé sortant de la chambre de réaction (3).

8. Appareil selon l'une quelconque des revendications précédentes, **caractérisé en ce que** ladite chambre de réaction (3) comporte une fenêtre en quartz (3c) permettant le passage de la lumière en son sein de manière à faciliter une réaction photocatalytique à l'intérieur de la chambre (3).

9. Procédé consistant à déterminer le degré de suppression d'agents polluants dans un mélange gazeux, **caractérisé en ce qu'**il comprend les étapes consistant à déposer un objet (P), revêtu d'une couche photocatalytique, à l'intérieur d'une chambre de réaction (3) d'un appareil selon l'une quelconque des revendications de 1 à 8, à introduire un mélange gazeux contrôlé dans l'appareil, à éclairer l'intérieur de la chambre de réaction (3) par rayonnement UV , à remplir ladite chambre de réaction (3) avec ledit mélange gazeux, à permettre au mélange gazeux sortant de la chambre de réaction (3) de passer à travers un appareil de test (13) pour la détermination continue et discontinue de la quantité d'agents polluants contenue dans le mélange gazeux quittant la chambre de réaction (3).

10. Procédé selon la revendication 9, **caractérisé en ce que** l'on provoque la sortie du mélange gazeux de la chambre de réaction (3) pour le faire passer à l'intérieur de l'appareil de test (13) à travers un circuit à cycle ouvert (14a) coopérant avec une unité de contrôle (16a) servant à l'évaluation continue des oxydes d'azote.

11. Procédé selon la revendication 9, **caractérisé en ce que** l'on provoque la sortie du mélange gazeux de la chambre de réaction (3) pour le faire passer à l'intérieur de l'appareil de test (13) à travers un circuit à cycle ouvert (14b) coopérant avec une unité de contrôle (16b) servant à l'évaluation continue des substances organiques volatiles.

12. Procédé selon la revendication 9, **caractérisé en ce que** l'on provoque la sortie du mélange gazeux de la chambre de réaction (3) pour le faire passer à l'intérieur de l'appareil de test (13) à travers un circuit à cycle fermé (15a) coopérant avec une unité de contrôle (16a) servant à l'évaluation discontinue des oxydes d'azote.

13. Procédé selon la revendication 9, **caractérisé en ce que** l'on provoque la sortie du mélange gazeux de la chambre de réaction (3) pour le faire passer à l'intérieur de l'appareil de test (13) à travers un circuit à cycle fermé (15b) coopérant avec une unité de contrôle (16b) servant à l'évaluation discontinue des substances organiques volatiles.

14. Procédé selon la revendication 12, **caractérisé en ce que**, avant d'effectuer l'évaluation discontinue des oxydes d'azote, une étape est prévue pour remplir un réservoir en forme de poche (22), disposé sur le circuit fermé (15a), ayant une quantité prédéterminée de mélange gazeux contrôle.

15. Procédé selon la revendication 11 ou 13, **caractérisé en ce qu'**il comprend l'étape consistant à provoquer la sortie du mélange gazeux de la chambre de réaction (3) pour le faire passer à travers un robinet d'échantillonnage (25) qui dirige de façon sélective un échantillon retiré de manière appropriée de masse gazeuze soit vers le circuit à cycle ouvert (14b) soit vers le circuit à cycle fermé (15b) pour l'analyse des quantités résiduelles de substances organiques volatiles.
